# EUROPEAN PATENT APPLICATION

(11) **EP 4 049 685 A1**
(43) Date of publication of application: **31.08.2022**
(21) Application number: 20894794.5
(22) Date of filing: 27.11.2020
(51) Int. Cl.: A61L 9/14, A61L 9/01

(54) **AROMATIC DEODORIZING DEVICE**

(30) Priority: 27.11.2019 JP 2019214164
(71) Applicant: Daikin Industries, Ltd., Osaka-shi, Osaka 530-8323 (JP)
(72) Inventor: AKIYAMA, Ryuuji, Osaka-shi, Osaka 530-8323 (JP); MIKI, Sanae, Osaka-shi, Osaka 530-8323 (JP); KOYAMA, Chika, Osaka-shi, Osaka 530-8323 (JP); TAKESHITA, Goushi, Osaka-shi, Osaka 530-8323 (JP); MITSUDA, Megumi, Nagoya-shi, Aichi 457-8530 (JP)
(74) Representative: Goddar, Heinz J.
(86) International application number: PCT/JP2020/044320
(87) International publication number: WO 2021/107126

(57) **Abstract**

An aromatic deodorizing device (10) generates a deodorant component that removes a target malodor. The aromatic deodorizing device (10) includes a sensor (60) that detects an index indicating an odor strength of the target malodor, a plurality of aromatic deodorants (C) of different types, and a switching unit (70) that changes the aromatic deodorant (C) that is to be a source of the deodorant component in accordance with a detection value of the sensor (60) .

## Description

### Technical Field

The present disclosure relates to an aromatic deodorizing device.

### Background Art

Conventionally, an aromatic deodorizing device that exhibits an aromatic deodorizing action by releasing an aromatic deodorant has been known. PTL 1 discloses an aromatic deodorizing device capable of adjusting the strength of an aromatic deodorizing action. This aromatic deodorizing device includes an evaporator, a fan, and an adjusting mechanism in a case. The fan blows air toward the evaporator. The adjusting mechanism controls the manner in which air is blown against the evaporator. In the aromatic deodorizing device of PTL 1, the evaporation amount of an aromatic deodorant is controlled by controlling the manner in which air is blown from the fan against the evaporator.

### Citation List

### Patent Literature

PTL 1: Japanese Unexamined Patent Application Publication No. 2004-65617

### Summary of Invention

### Technical Problem

However, the aromatic deodorizing device of PTL 1 does not control the amount of evaporation of the aromatic deodorant in consideration of the intensity of a target malodor. Therefore, when the intensity of the target malodor changes, the amount of generated aromatic deodorant may no longer be appropriate, and the unpleasantness felt by a person with respect to the target malodor may increase.

An object of the present disclosure is to suppress an increase in unpleasantness felt by a person caused by a change in intensity of a target malodor.

### Solution to Problem

A first aspect of the present disclosure is an aromatic deodorizing device (10) configured to generate a deodorant component that removes a target malodor. The aromatic deodorizing device (10) includes a sensor (60) configured to detect an index indicating an odor strength of the target malodor, a plurality of aromatic deodorants (C) of different types, and a switching unit (70) configured to change the aromatic deodorant (C) that is to be a source of the deodorant component in accordance with a detection value of the sensor (60).

In the first aspect, the aromatic deodorant (C) to be generated is changed by the switching unit (70) in accordance with the odor strength detected by the sensor (60). Therefore, it is possible to suppress an increase in unpleasantness felt by a person caused by a change in intensity of the target malodor.

A second aspect of the present disclosure is the aromatic deodorizing device according to the first aspect, in which the switching unit (70) is configured to change the aromatic deodorant (C) that is to be the source of the deodorant component so as to suppress a decrease in a pleasantness/unpleasantness level of the target malodor.

In the second aspect, the aromatic deodorant (C) is changed by the switching unit (70) so as to suppress a decrease in the pleasantness/unpleasantness level of the target malodor. Therefore, it is possible to suppress an increase in unpleasantness felt by a person caused by a change in intensity of the target malodor.

A third aspect of the present disclosure is the aromatic deodorizing device according to the first aspect or the second aspect, in which the plurality of aromatic deodorants (C) include a first aromatic deodorant (C1) and a second aromatic deodorant (C2), and in which the switching unit (70) is configured to cause the first aromatic deodorant (C1) to be the source of the deodorant component when the detection value of the sensor (60) is less than or equal to a first value, and is configured to cause the second aromatic deodorant (C2) to be the source of the deodorant component when the detection value of the sensor (60) is greater than a second value that is greater than or equal to the first value.

In the third aspect, since the aromatic deodorant (C) that generates a deodorant component having a high effect on the detection value detected by the sensor (60) is selected by the switching unit (70), it is possible to suppress an increase in unpleasantness felt by a person caused by a change in intensity of the target malodor.

A fourth aspect of the present disclosure is the aromatic deodorizing device according to the third aspect, in which the first aromatic deodorant (C1) consists of carvone, citral, trans-2-hexenal, benzaldehyde, and cinnamaldehyde, and the second aromatic deodorant (C2) consists of limonene, citral, citronellal, and cinnamaldehyde.

In the fourth aspect, the first aromatic deodorant (C1) is capable of removing a target malodor having a relatively low malodor odor strength, and the second aromatic deodorant (C2) is capable of removing a target malodor having a relatively high malodor odor strength. Therefore, it is possible to suppress an increase in unpleasantness felt by a person caused by a change in intensity of the target malodor.

### Brief Description of Drawings

[Fig. 1] Fig. 1 is a schematic overall structural view of a target space to which an aromatic deodorizing device according to an embodiment is applied.
[Fig. 2] Fig. 2 is a schematic structural view of the aromatic deodorizing device.
[Fig. 3] Fig. 3 is a block diagram of the aromatic deodorizing device.
[Fig. 4] Fig. 4 is a table showing samples used in an evaluation test of a deodorizing effect.
[Fig. 5] Fig. 5 is a table showing a nine-level pleasantness/unpleasantness indication method for use in the evaluation test of the deodorizing effect.
[Fig. 6] Fig. 6 is a table showing a six-level odor intensity indication method for use in the evaluation test of the deodorizing effect.
[Fig. 7] Fig. 7 is a graph showing changes in odor intensity and pleasantness/unpleasantness level when a fragrance material is added to n-valeric acid.

### Description of Embodiments

### <<Embodiments>>

An embodiment is described.

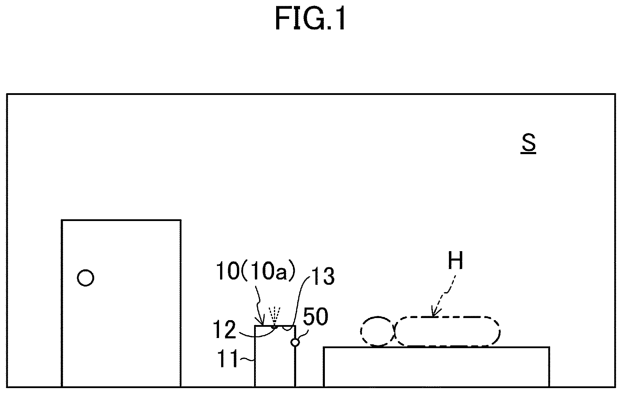

As shown in Fig. 1, an aromatic deodorizing device (10) of the present disclosure is disposed in an indoor space (S) of a nursing facility for the elderly, a hospital, or the like. A person (H) who produces a nursing environment odor, which becomes a target malodor, exists in the indoor space (S). In the example of Fig. 1, the person (H) is a source of a malodor. Here, the nursing environment odor is a malodor generated from deteriorated sebum, deteriorated urine, or the like resulting from the life of an elderly person, a sick person, or a handicapped person. The nursing environment odor contains n-valeric acid and acetaldehyde.

The aromatic deodorizing device (10) of the present disclosure sprays an aromatic deodorant (C) into the indoor space (S) to generate a deodorant component. The aromatic deodorant (C) is a source of a predetermined deodorant component. The aromatic deodorant (C) removes a target malodor by the deodorant component contained in the aromatic deodorant. The aromatic deodorant (C) is a fragrance material. The aromatic deodorant (C) contains a fragrance component.

The aromatic deodorizing device (10) of the present disclosure includes a first aromatic deodorant (C1) and a second aromatic deodorant (C2). In other words, the aromatic deodorizing device (10) of the present disclosure includes a plurality of aromatic deodorants (C) of different types.

The fragrance components contained in the first aromatic deodorant (C1) of the present embodiment are carvone, citral, trans-2-hexenal, benzaldehyde, and cinnamaldehyde. In other words, the fragrance components of the first aromatic deodorant (C1) are five components, carvone, citral, trans-2-hexenal, benzaldehyde, and cinnamaldehyde.

The fragrance components contained in the second aromatic deodorant (C2) of the present embodiment are limonene, citral, citronellal, and cinnamaldehyde. In other words, the fragrance components of the second aromatic deodorant (C2) are four components, limonene, citral, citronellal, and cinnamaldehyde.

The mixing amount of the fragrance components of the aromatic deodorant (C) is selected as appropriate in accordance with components to be mixed other than the components above, the deodorizing effect on the target malodor, and the like, and is not limited.

The malodor is sensorily removed by the aromatic deodorant (C) sprayed by the aromatic deodorizing device (10). Here, "sensory deodorization" includes "masking" and "modulation". In the "masking", the sensitivity to the malodor is reduced by a relatively highly intense smell of the fragrance components. In the "modulation", by mixing the fragrance components with the malodor, the quality of the smell affecting a person's sense of smell is changed, and the smell is hardly perceived as a malodor. The aromatic deodorizing device (10) of the present embodiment modulates the malodor by spraying a relatively small amount of the aromatic deodorant (C) in accordance with the malodor.

### -Aromatic Deodorizing Device-

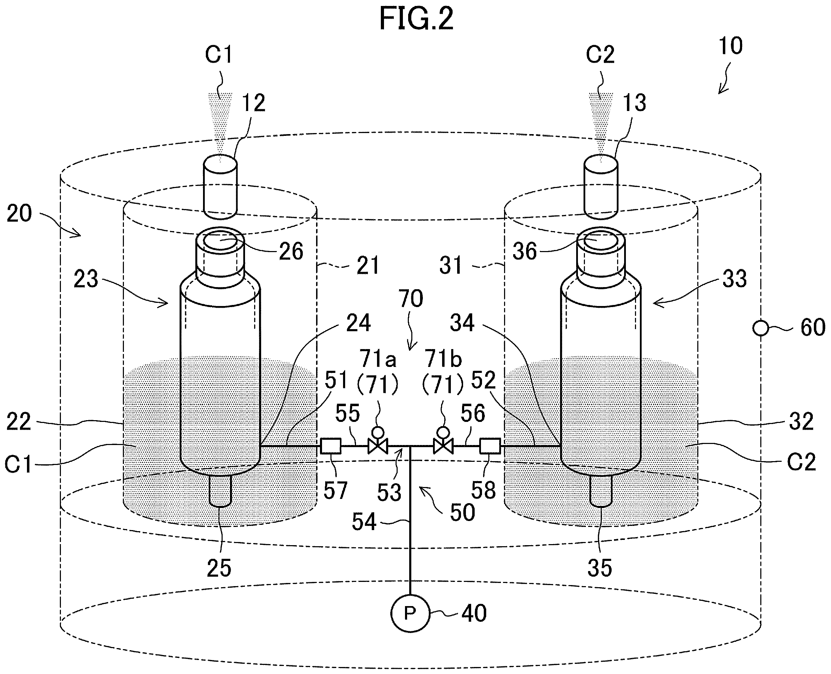

As shown in Fig. 2, the aromatic deodorizing device (10) includes a sprayer body (10a) and a sensor (60) integrally attached to the sprayer body (10a). The sprayer body (10a) includes a casing (11), and a spray unit (20), the sensor (60), and a switching unit (70), which are housed inside the casing (11).

### <Casing>

The casing (11) is hollow. The casing (11) has a first supply port (12) through which the atomized first aromatic deodorant (C1) is supplied to the indoor space (S) and a second supply port (13) through which the atomized second aromatic deodorant (C2) is supplied to the indoor space (S).

### <Spray Unit>

The spray unit (20) includes a first spray cartridge (21), a second spray cartridge (31), an air pump (40), an air flow path (50), and a switching mechanism (71).

The first spray cartridge (21) is detachably attached to the inside of the casing (11). The first spray cartridge (21) includes a first tank (22) and a first two-fluid nozzle (23). The first aromatic deodorant (C1) is stored in the first tank (22). The first two-fluid nozzle (23) constitutes an atomizing mechanism for atomizing the first aromatic deodorant (C1) in the first tank (22). Specifically, the first two-fluid nozzle (23) atomizes a liquid membrane of the first aromatic deodorant (C1) by a shearing force of an airflow.

The first two-fluid nozzle (23) has an inflow port (24) through which air flows in, a suction port (25) through which the first aromatic deodorant (C1) is sucked, and a spray port (26) through which the atomized first aromatic deodorant (C1) is discharged. The inflow port (24) is formed in the peripheral wall of the body of the first two-fluid nozzle (23). The suction port (25) is formed at the lower end of the body of the first two-fluid nozzle (23). The spray port (26) is formed at the upper end of the body of the first two-fluid nozzle (23).

The structure of the second spray cartridge (31) is the same as that of the first spray cartridge (21). The second aromatic deodorant (C2) is stored in a second tank (32) of the second spray cartridge (31).

The air flow path (50) is connected to a discharge portion of the air pump (40). The air pump (40) sucks the air in the indoor space (S) and discharges the sucked air to the air flow path (50). The air pump (40) supplies the air to the first two-fluid nozzle (23) of the first spray cartridge (21) and a second two-fluid nozzle (33) of the second spray cartridge (31).

The air flow path (50) includes a first inner flow path (51), a second inner flow path (52), and an outer flow path (53). The first inner flow path (51) is disposed inside the first spray cartridge (21). The second inner flow path (52) is disposed inside the second spray cartridge (31). The outer flow path (53) is disposed outside the first spray cartridge (21) and the second spray cartridge (31).

The outer flow path (53) includes a main flow path (54), a first branch path (55), and a second branch path (56). An inflow end of the main flow path (54) is connected to the air pump (40). An outflow end of the main flow path (54) is connected to an inflow end of the first branch path (55) and an inflow end of the second branch path (56). In other words, the main flow path (54) branches into the first branch path (55) and the second branch path (56).

An outflow end of the first branch path (55) is connected to an inflow end of a first fluid joint (57). An outflow end of the first fluid joint (57) is connected to an inflow end of the first inner flow path (51). In other words, the first branch path (55) and the first inner flow path (51) are connected to each other via the first fluid joint (57). The first fluid joint (57) is disposed outside the first spray cartridge (21). An outflow end of the first inner flow path (51) is connected to the inflow port (24) of the first two-fluid nozzle (23).

An outflow end of the second branch path (56) is connected to an inflow end of a second fluid joint (58). An outflow end of the second fluid joint (58) is connected to an inflow end of the second inner flow path (52). In other words, the second branch path (56) and the second inner flow path (52) are connected to each other via the second fluid joint (58). The second fluid joint (58) is disposed outside the second spray cartridge (31). An outflow end of the second inner flow path (52) is connected to an inflow port (34) of the second two-fluid nozzle (33).

The switching mechanism (71) is disposed in the outer flow path (53). In the present embodiment, the switching mechanism (71) includes a first electromagnetic valve (71a) and a second electromagnetic valve (71b). The first electromagnetic valve (71a) is disposed in the first branch path (55). The second electromagnetic valve (71b) is disposed in the second branch path (56).

### <Sensor>

The sensor (60) detects an index indicating the odor strength of a malodor (in the present embodiment, a nursing environment odor) generated in the indoor space (S). The so-called index indicating the odor strength of the malodor includes the concentration of a substance with the malodor, the odor concentration of the malodor, and the odor intensity corresponding to the concentration of the substance with the malodor. The sensor (60) of the present embodiment detects the odor intensity of a malodor corresponding to a nursing environment odor.

The sensor (60) of the present embodiment is provided integrally with the sprayer body (10a) (see Figs. 1 and 2). Strictly speaking, the sensor (60) is disposed in the casing (11) so as to be exposed to the indoor space (S).

### <Switching Unit>

The switching unit (70) of the present embodiment changes the first aromatic deodorant (C1) and the second aromatic deodorant (C2) in accordance with the detection value of the sensor (60). The switching unit (70) includes the switching mechanism (71) and a control unit (80).

The switching mechanism (71) of the present example includes the first electromagnetic valve (71a) and the second electromagnetic valve (71b). When the first aromatic deodorant (C1) is generated from the aromatic deodorizing device (10), the first electromagnetic valve (71a) is opened and the second electromagnetic valve (71b) is closed. Thus, air supplied from the air pump (40) is supplied to the first two-fluid nozzle (23) of the first spray cartridge (21), while air is not supplied to the second spray cartridge (31). When the second aromatic deodorant (C2) is generated from the aromatic deodorizing device (10), the second electromagnetic valve (71b) is opened and the first electromagnetic valve (71a) is closed. Thus, air supplied from the air pump (40) is supplied to the second two-fluid nozzle (33) of the second spray cartridge (31), while air is not supplied to the first spray cartridge (21). In other words, the aromatic deodorant (C) sprayed from the aromatic deodorizing device (10) is changed by controlling the opening and closing of the first electromagnetic valve (71a) and the second electromagnetic valve (71b).

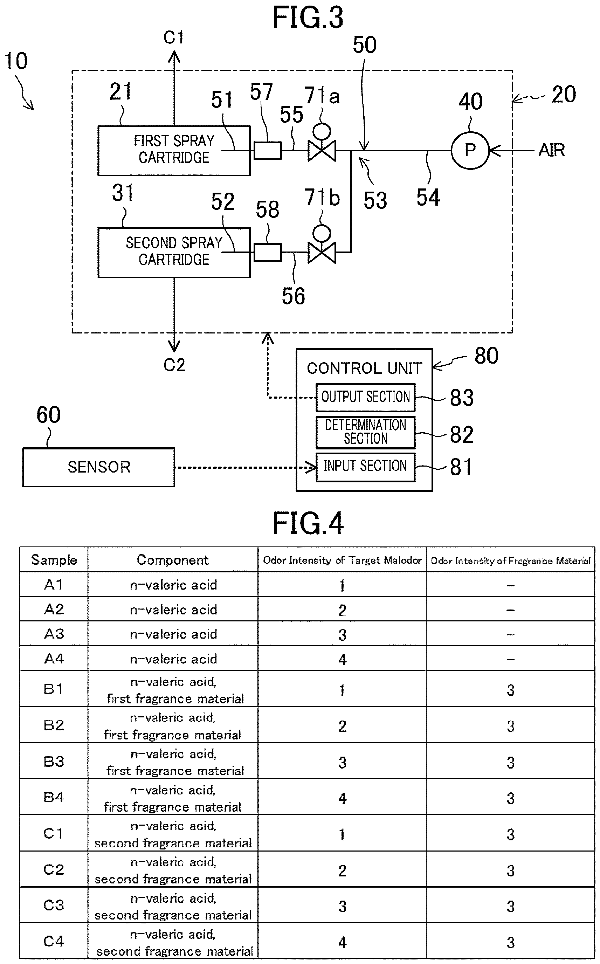

As shown in Fig. 3, the control unit (80) includes an input section (81), a determination section (82), and an output section (83). A signal indicating the odor intensity of a malodor detected by the sensor (60) is input to the input section (81). The determination section (82) performs determination for changing the aromatic deodorant (C) so as to suppress a decrease in the pleasantness/unpleasantness level of the target malodor. Specifically, the determination section (82) determines whether the odor intensity of the malodor input to the input section (81) exceeds a predetermined value.

The output section (83) outputs to the spray unit (20) a control signal for spraying the first aromatic deodorant (C1) when the odor intensity of the malodor is less than or equal to a predetermined first value as a result of the determination by the determination section (82). The output section (83) outputs to the spray unit (20) a control signal for spraying the second aromatic deodorant (C2) when the odor intensity of the malodor is larger than a predetermined second value as a result of the determination by the determination section (82). In the present embodiment, the first value and the second value are set to be equal values.

The control unit (80) includes a microcomputer mounted on a control board, and a memory device (specifically, a semiconductor memory) that stores software for operating the microcomputer.

The spray amount is adjusted by adjusting the time interval between intermittent operations of the air pump (40), the discharge flow rate of the air pump (40), the opening degree of the air flow path (50), or the like.

### -Driving Operation-

An operation of the aromatic deodorizing device (10) is described. The aromatic deodorizing device (10) performs a first operation and a second operation. The first operation is an operation of generating a deodorant component from the first aromatic deodorant (C1). The second operation is an operation of generating a deodorant component from the second aromatic deodorant (C2). The aromatic deodorizing device (10) switches between the first operation and the second operation in accordance with the detection value of the sensor (60). The first operation and the second operation are described in detail.

### <First Operation>

When a malodor is generated in the indoor space (S), a person in the space feels unpleasant. After the generation of the malodor, the odor intensity of the malodor is detected by the sensor (60) and is input to the input section (81) of the control unit (80). When a detection value is input to the input section (81), the determination section (82) of the control unit (80) determines whether the input odor intensity of the malodor exceeds a predetermined value. As a result of the determination, if the detection value is less than or equal to the predetermined first value, a control signal for spraying the first aromatic deodorant (C1) is output from the output section (83) of the control unit (80) to the spray unit (20), and the aromatic deodorizing device (10) performs a spraying operation.

In the above spraying operation, the air pump (40) is turned on, the first electromagnetic valve (71a) is open, and the second electromagnetic valve (71b) is closed. Air conveyed by the air pump (40) passes through the main flow path (54) and the first branch path (55) of the outer flow path (53), and is introduced into the first spray cartridge (21). In the first two-fluid nozzle (23), the first aromatic deodorant (C1) is atomized by the flow of the air. The atomized first aromatic deodorant (C1) is supplied into the indoor space (S) through the spray port (26) and the first supply port (12).

### <Second Operation>

As a result of the determination by the control unit (80), if the detection value is larger than the predetermined second value, a control signal for spraying the second aromatic deodorant (C2) is output from the output section (83) of the control unit (80) to the spray unit (20), and the aromatic deodorizing device (10) performs a spraying operation.

In the above spraying operation, the air pump (40) is turned on, the first electromagnetic valve (71a) is closed, and the second electromagnetic valve (71b) is open. Air conveyed by the air pump (40) passes through the main flow path (54) and the second branch path (56) of the outer flow path (53), and is introduced into the second spray cartridge (31). In the second two-fluid nozzle (33), the second aromatic deodorant (C2) is atomized by the flow of the air. The atomized second aromatic deodorant (C2) is supplied into the indoor space (S) through a spray port (36) and the second supply port (13).

### -Sensory Deodorizing Effect of Aromatic Deodorant According to Embodiment-

Evaluation results of a sensory deodorizing effect of the first aromatic deodorant (hereafter, also referred to as a "first fragrance material") and the second aromatic deodorant (hereafter, also referred to as a "second fragrance material") according to the embodiment are described.

### (1) Fragrance Components of Aromatic Deodorant

The fragrance components contained in the first fragrance material for use in an evaluation test are carvone, citral, trans-2-hexenal, benzaldehyde, and cinnamaldehyde.

The fragrance components contained in the second fragrance material for use in the evaluation test are limonene, citral, citronellal, and cinnamaldehyde.

### (2) Evaluation Test of Deodorizing Effect

In this test, samples were first put into respective bags, and subjects smelled an odor in each bag. Next, the subjects sensorily evaluated the pleasantness/unpleasantness level of the odor in each bag.

Target malodors of this test are n-valeric acid. The samples of this test are samples A1 to A4, B1 to B4, and C1 to C4 having respective odors of twelve types as shown in Fig. 4. Specifically, the samples A1 to A4 each have only the target malodor, and the odor intensity of the sample A1 is adjusted to 1, the odor intensity of the sample A2 is adjusted to 2, the odor intensity of the sample A3 is adjusted to 3, and the odor intensity of the sample A4 is adjusted to 4. The samples B1 to B4 each have a mixed odor obtained by adding a first fragrance material adjusted to have an odor intensity of 3 to a corresponding one of the samples A1 to A4. The samples C1 to C4 each have a mixed odor obtained by adding a second fragrance material adjusted to have an odor intensity of 3 to a corresponding one of the samples A1 to A4.

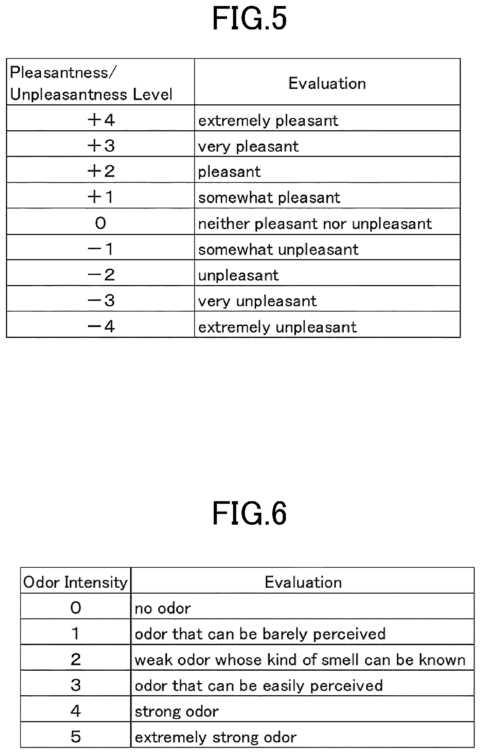

The number of subjects is 15. The subjects evaluated the pleasantness/unpleasantness level based on evaluating criteria of a nine-level pleasantness/unpleasantness indication method shown in Fig. 5. Then, an average value of the evaluation by each subject was determined. Note that the odor intensities of the target malodors and the fragrances were adjusted using a six-level odor intensity indication method shown in Fig. 6.

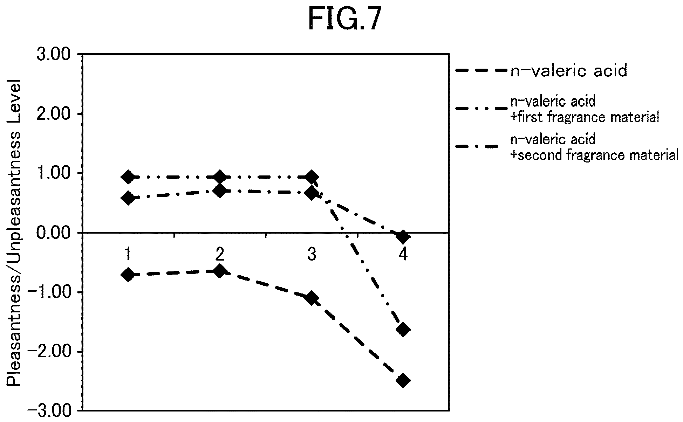

The results of the samples having the same target-malodor odor intensity were compared. As shown in Fig. 7, when the target-malodor odor intensity was 1 to 3, the pleasantness/unpleasantness level of the samples B1 to B3 was higher than the pleasantness/unpleasantness level of the samples C1 to C3. On the other hand, when the target-malodor odor intensity was 4, the pleasantness/unpleasantness level of the sample B4 was lower than the pleasantness/unpleasantness level of the sample C4.

Therefore, it was confirmed that when the target-malodor odor intensity was 1 to 3, the first fragrance material had a higher deodorizing effect on the target malodor than the second fragrance material, and when the target odor intensity was 4, the second fragrance material had a higher deodorizing effect on the target malodor than the first fragrance material.

Consequently, in order to remove the target malodor, the first fragrance material is used when the target-malodor odor intensity is 1 to 3, and the second fragrance material is used when the target-malodor odor intensity is 4, as a result of which it is possible to suppress an increase in unpleasantness caused by a change in the target odor.

### -Feature (1) of Embodiment-

The aromatic deodorizing device (10) of the present embodiment generates a deodorant component that removes a target malodor. The aromatic deodorizing device includes a sensor (60) that detects an index indicating the odor strength of a target malodor, a plurality of aromatic deodorants (C) of different types, and a switching unit (70) that changes the aromatic deodorant (C) that is to be a source of the deodorant component in accordance with a detection value of the sensor (60).

In the aromatic deodorizing device (10) of the present embodiment, the aromatic deodorizer (C) to be generated is changed by the switching unit (70) in accordance with the odor strength detected by the sensor (60). Therefore, it is possible to suppress an increase in unpleasantness felt by a person caused by a change in intensity of the target malodor.

### -Feature (2) of Embodiment-

The switching unit (70) of the aromatic deodorizing device (10) of the present embodiment changes the aromatic deodorant (C) that is to be the source of the deodorant component so as to suppress a decrease in a pleasantness/unpleasantness level of the target malodor.

In the aromatic deodorizing device (10) of the present embodiment, the aromatic deodorizer (C) is changed by the switching unit (70) so as to suppress a decrease in the pleasantness/unpleasantness level of the target malodor. Therefore, it is possible to suppress an increase in unpleasantness felt by a person caused by a change in intensity of the target malodor.

### -Feature (3) of Embodiment-

The aromatic deodorants (C) of the aromatic deodorizing device (10) of the present embodiment includes a first aromatic deodorant (C1) and a second aromatic deodorant (C2), and the switching unit (70) causes the first aromatic deodorant (C1) to be the source of the deodorant component when the detection value of the sensor (60) is less than or equal to a first value, and causes the second aromatic deodorant (C2) to be the source of the deodorant component when the detection value of the sensor (60) is greater than a second value that is greater than or equal to the first value.

In the aromatic deodorizing device (10) of the present embodiment, since the aromatic deodorizer (C) that generates a deodorant component having a high effect on the detection value detected by the sensor (60) is selected by the switching unit (70), it is possible to suppress an increase in unpleasantness felt by a person caused by a change in intensity of the target malodor.

### -Feature (4) of Embodiment-

In the aromatic deodorizing device (10) of the present embodiment, the first aromatic deodorant (C1) consists of carvone, citral, trans-2-hexenal, benzaldehyde, and cinnamaldehyde, and the second aromatic deodorant (C2) consists of limonene, citral, citronellal, and cinnamaldehyde.

In the aromatic deodorizing device (10) of the present embodiment, the first aromatic deodorant (C1) is capable of removing a target malodor having a relatively low malodor odor strength, and the second aromatic deodorant (C2) is capable of removing a target malodor having a relatively high malodor odor strength. Therefore, it is possible to suppress an increase in unpleasantness felt by a person caused by a change in strength of the target malodor.

The target malodor in the aromatic deodorizing device (10) of the present embodiment is a nursing environment odor. The nursing environment odor contains n-valeric acid. From the results of the evaluation test of the deodorizing effect, it was confirmed that the first aromatic deodorant (C1) had a high deodorizing effect on target malodors having a relatively low target-malodor odor intensity of 1 to 3, and the second aromatic deodorant (C2) had a high deodorizing effect on target malodors having a relatively high target-malodor odor intensity of 4. Therefore, by switching the aromatic deodorant (C) to be generated to the first aromatic deodorant (C1) or the second aromatic deodorant (C2) in accordance with the detection value of the sensor (60), it is possible to suppress an increase in unpleasantness felt by a person caused by a change in intensity of the target malodor.

### <<Other Embodiments>>

The embodiment above may feature the following structures.

The target malodor of the embodiment above merely needs to contain n-valeric acid, and may be an odor other than a nursing environment odor. Examples of the target malodor include a shoe-rack odor and a pet odor.

The aromatic deodorizing device (10) of the embodiment above may include two or more types of aromatic deodorants (C) .

The aromatic deodorants (C) of the embodiment above may contain a diluent, such as ethanol or water.

The aromatic deodorizing device (10) of the embodiment above merely needs to be one that generates a deodorant component, and need not be of a type that sprays the aromatic deodorants (C) into a space. For example, the aromatic deodorants (C) may be directly sprayed on the malodor source or may be evaporated in the space.

In the aromatic deodorizing device (10) of the embodiment above, the atomizing mechanism may be of another type, such as a piezoelectric type, an electrostatic spray type, or an ultrasonic type.

Although the switching mechanism (71) of the embodiment above uses the first electromagnetic valve (71a) and the second electromagnetic valve (71b), it may be a three-way valve.

In the embodiment above, although the first value and the second value are set to be the same value, the second value may be greater than the first value. This makes it possible to suppress an increase in the number of times the aromatic deodorant (C) to be generated is switched when the detection value of the sensor (60) changes at about a threshold value within a short time.

The aromatic deodorizing device (10) of the embodiment above may be applied to an air cleaner, an air conditioner, a ventilator, a humidifier, a dehumidifier, or the like.

Although the embodiments and modifications have been described above, it will be understood that various changes in form and detail can be made without departing from the spirit and scope of the claims. The embodiments and the modifications above may be combined or replaced as appropriate as long as the object functions of the present disclosure are not impaired.

### Industrial Applicability

As described above, the present disclosure is useful for an aromatic deodorizing device.

### Reference Signs List

- C1: first aromatic deodorant
- C2: second aromatic deodorant
- 10: aromatic deodorizing device
- 20: spray unit
- 21: first spray cartridge
- 31: second spray cartridge
- 40: air pump
- 50: air flow path
- 60: sensor
- 70: switching unit
- 71: switching mechanism
- 80: control unit

## Claims

1. An aromatic deodorizing device (10) configured to generate a deodorant component that removes a target malodor, the aromatic deodorizing device (10) comprising:
a sensor (60) configured to detect an index indicating an odor strength of the target malodor;
a plurality of aromatic deodorants (C) of different types; and
a switching unit (70) configured to change the aromatic deodorant (C) that is to be a source of the deodorant component in accordance with a detection value of the sensor (60) .

2. The aromatic deodorizing device according to claim 1, wherein the switching unit (70) is configured to change the aromatic deodorant (C) that is to be the source of the deodorant component so as to suppress a decrease in a pleasantness/unpleasantness level of the target malodor.

3. The aromatic deodorizing device according to claim 1 or claim 2,
wherein the plurality of aromatic deodorants (C) include a first aromatic deodorant (C1) and a second aromatic deodorant (C2), and
wherein the switching unit (70) is configured to cause the first aromatic deodorant (C1) to be the source of the deodorant component when the detection value of the sensor (60) is less than or equal to a first value, and is configured to cause the second aromatic deodorant (C2) to be the source of the deodorant component when the detection value of the sensor (60) is greater than a second value that is greater than or equal to the first value.

4. The aromatic deodorizing device according to claim 3,
wherein the first aromatic deodorant (C1) consists of carvone, citral, trans-2-hexenal, benzaldehyde, and cinnamaldehyde, and
wherein the second aromatic deodorant (C2) consists of limonene, citral, citronellal, and cinnamaldehyde.
